# EUROPEAN PATENT SPECIFICATION

(11) **EP 3 223 988 B1**
(45) Date of publication and mention of the grant of the patent: **30.12.2020**
(21) Application number: 15798473.3
(22) Date of filing: 25.11.2015
(51) Int. Cl.: B23K 9/00, B23K 9/12, B23K 9/167, B23K 9/29

(54) **WELDING TOOL FOR PERFORMING SMAW OR MIG WELD WITH MAINTENANCE OF A CONSTANT DISTANCE BETWEEN THE ELECTRODE AND THE WELD AREA**
SCHWEISSWERKZEUG ZUR DURCHFÜHRUNG VON SMAW- ODER MIG-SCHWEISSEN MIT EINHALTUNG EINES KONSTANTEN ABSTANDES ZWISCHEN ELEKTRODE UND SCHWEISSBEREICH
OUTIL DE SOUDAGE POUR LA RÉALISATION DE SOUDAGE SMAW OU MIG EN MAINTENANT UNE DISTANCE CONSTANTE ENTRE L'ÉLECTRODE ET LA ZONE DE SOUDURE

(30) Priority: 27.11.2014 IT MI20142040
(43) Date of publication of application: 04.10.2017
(73) Proprietor: Nuovo Pignone S.r.l., 50127 Florence (IT)
(72) Inventor: CHIARI, Francescosaverio, I-50127 Florence (IT); MILAZZO, Mario, I-50127 Florence (IT); MAGNASCO, Marco, I-50127 Florence (IT); STEFANINI, Cesare, I-50127 Florence (IT); INGLESE, Francesco, I-50127 Florence (IT)
(74) Representative: BRP Renaud & Partner mbB Rechtsanwälte Patentanwälte Steuerberater
(86) International application number: PCT/EP2015/077591
(87) International publication number: WO 2016/083429

(56) References cited:
- CA-A1- 2 485 302
- JP-A- 2001 129 666
- US-A- 3 030 489
- US-A1- 2009 276 930
- US-A1- 2012 180 180
- US-B1- 6 242 711

## Description

The subject matter of the present disclosure relates to a welding tool and an arc welding kit, namely, a set of tools that is used to perform a manual arc welding operation.

CA 2 485 302 A1, basis for the preamble of claim 1, discloses a power cable. Heads can be interchanged without tools and without the hazard of an exposed connector at the end of the cable.

A known arc welding kit comprises a welding mask and a welding tool. The welding tool comprises an electrode. During welding operations, an electric arc develops between the electrode and the welding area.

In a first and second type of arc welding, the SMAW (Shielded Metal Arc Welding) and the MIG (Metal Inert Gas), the electrode itself melts due to the heat developed by the electric arc, thus becoming the filler material in the weld.

In a third type of arc welding, the TIG (Tungsten Inert Gas), the electrode is solid, and the filler material is provided separately.

With more detail, the kit comprises a set of sensors which can detect the main operating parameters of a welding process, namely the voltage (V), the current (A), the welding speed (W) and their combination to calculate the heat input. The welding mask can be provided with a display device so that these parameters can be shown to a welder, thereby providing him with a possibility of correcting the welding in real time. An example of this welding mask is the one shown in the document US 6242711 B1.

A disadvantage of the known welding kit is that it merely provides the welder with the welding parameters. However, this does not guarantee that the welder is able to adapt and correct a welding that is being performed improperly. In other words, the welding operation itself still relies heavily on the manual skill of the welder. This is particularly true with respect to the welding voltage, since it is mainly determined by the distance of the electrode from the weld area, thus the work piece.

### SUMMARY

A welding tool according to the present invention is defined in claim 1.

An arc welding kit comprising such welding tool is further defined in claim 8.

Further embodiments are defined in the dependent claims.

The welding tool of the present invention comprises a main body. A handle is attached to the main body, and is configured to be held by a welder. An electrode is also provided, and is in particular inserted into the main body. An adjusting device is associated with the electrode for moving it forward/backward with respect to the main body. A control unit is connected to the adjusting device. The control unit is configured to act on the adjusting device for maintaining a substantially constant distance between the electrode and a weld area. The electrode is, according to the present invention, consumable for performing a SMAW or a MIG weld.

The welding tool is able to compensate the variation of the distance of the electrode from the weld area. Therefore, even if the welder is somewhat less skilled, the welding can still be performed adequately, as tests have shown. Also, this system is able to compensate small distance variation due to the physiologic tremor of the hand even better than a skilled welder, thus leading to an overall quality improvement of the weld.

The kit according to the present invention further comprises a welding mask. A welding velocity sensor is attached on the welding mask. The welding velocity sensor is configured to detect a welding velocity and to emit a welding velocity signal representing a value of the welding velocity. The control unit comprises a processing module which is configured to compute a velocity difference between the welding velocity and a target velocity value for the welding velocity. The processing module is also configured to emit a velocity difference signal representing the result of the velocity difference. The welding mask comprises a visualization device which is configured to acquire the velocity difference signal and to show a representation of the velocity difference and of consequent heat input difference to a welder. Advantageously, in this way the welder has also a feedback on the welding velocity which, in addition to a constant current and to a stable voltage, greatly improves the overall quality of the weld allowing to respect target heat input.

Further details and specific embodiments will refer to the attached drawings, in which:
- Figure 1 is a schematic representation of an arc welding kit according to an embodiment of the present invention;
- Figure 2 is a side sectional view of a welding tool according to an embodiment of the present invention;
- Figure 3 is a front sectional view of the welding tool of figure 2;
- Figure 4 is a side sectional view of a welding tool not covered by the present invention;
- Figure 5 is a side sectional view of a component of the kit of figure 1;
- Figure 6 is a top sectional view of the component of figure 5;
- Figure 6a is an enlarged view of a detail of figure 6;

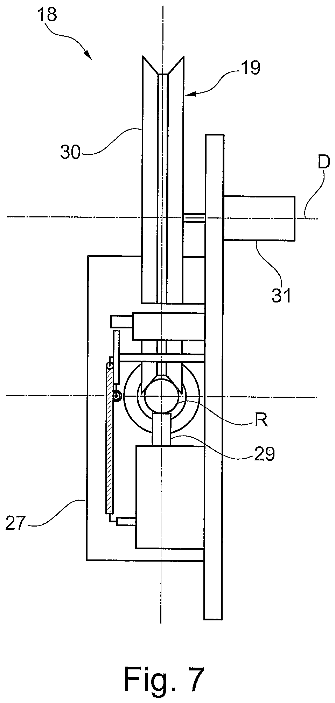

- Figure 7 is a front sectional view of the component of figures 5 and 6;
- Figure 8 is a front view of a further component of the kit of figure 1; and

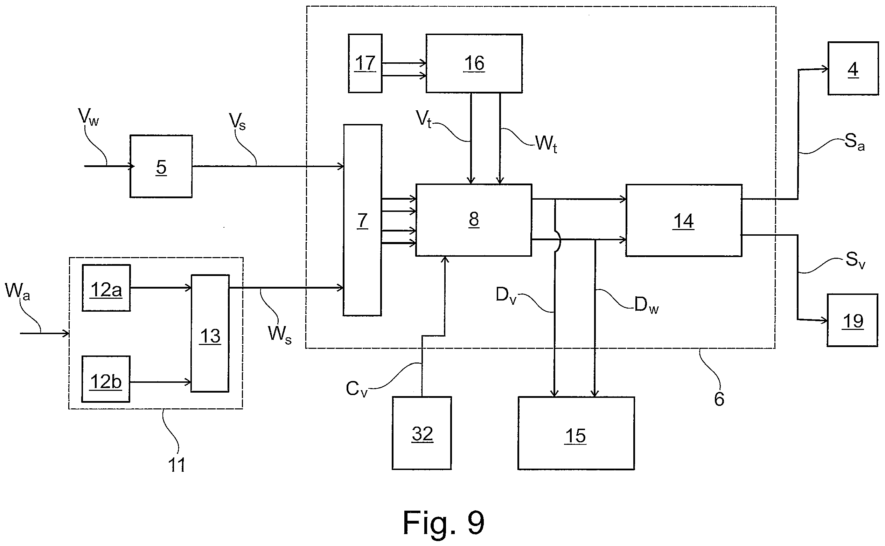

- Figure 9 is a schematic representation of the functioning of the kit of figure 1.

### DETAILED DESCRIPTION

The following description of exemplary embodiments refer to the accompanying drawings. The same reference numbers in different drawings identify the same or similar elements. The following detailed description does not limit the invention. Instead, the scope of the invention is defined by the appended claims.

With reference to the attached drawings, with the number 1 is indicated an arc welding kit according to an embodiment of the present invention. The welding kit 1 comprises a welding tool 2, which is configured to be held by a welder.

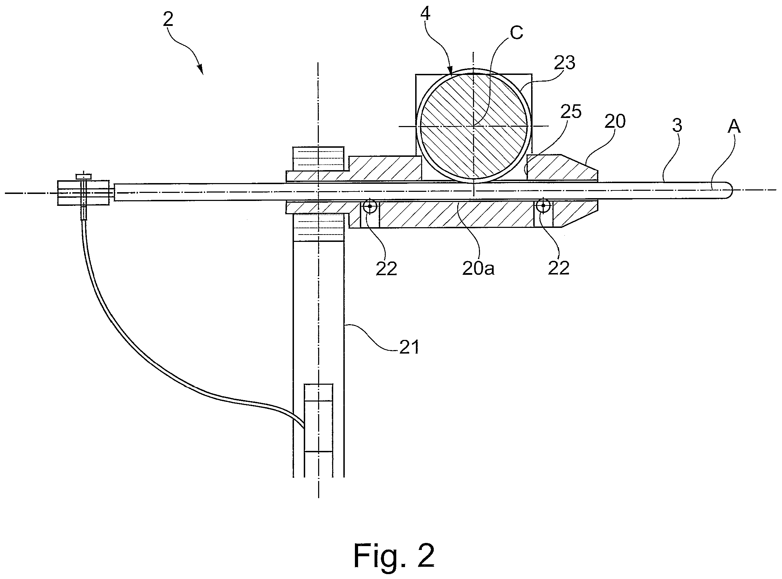

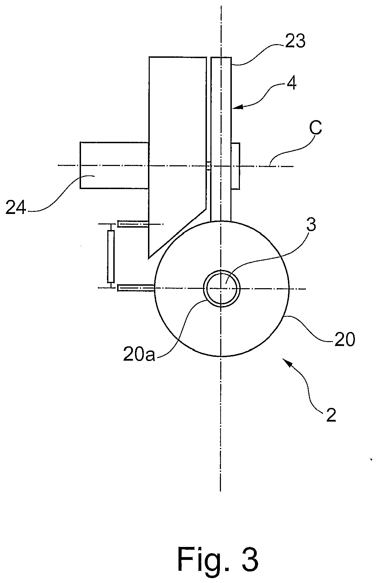

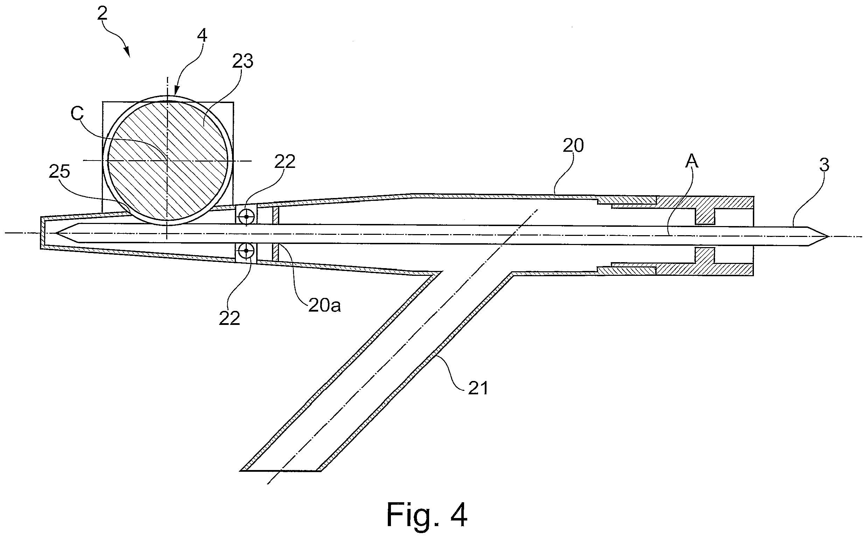

The welding tool 2 comprises an electrode 3, which is used, according to the present invention, to perform a SMAW (shielded metal arc weld) or a MIG (Metal Inert Gas), shown in figures 2 and 3, the electrode 3 is consumable. In other words, according to the present invention, the electrode 3 becomes the filler material of the weld. In an example not covered by the present invention, as shown in figure 4, the electrode 3 is non-consumable, thus it is used to perform a TIG (Tungsten Inert Gas) weld.

With additional details, the welding tool 2 comprises a main body 20, configured to support the electrode 3. The main body 20 is preferably axial-symmetric, and develops mainly along a longitudinal axis "A". A handle 21 for the welder supports the main body 20.

The main body 20 has a seat 20a in which the electrode 3 is installed. As shown in figures 2 and 4, the welding tool 2 is provided with bearings 22, which are attached to the main body and located in proximity of the seat 20, so that they can support the electrode 3 and allow it to move forward and backward. In other words, the electrode 3 can move forward and backward inside the seat 20a by sliding on the bearings 22.

Also, the welding tool 2 comprises an adjusting device 4 associated with the electrode 3, in order to move the electrode forward/backward with respect to the main body 20. The adjusting device 4 comprises a wheel 23 having a central axis "C" disposed transversally and preferably perpendicularly, to the longitudinal axis "A" of the electrode 3, that is parallel to the axis of the main body 20. Indeed, the main body 20 is provided with a port 25 in which the wheel 24 is inserted.

In operation, the rim of the wheel 23 is in contact with the electrode 3 so that the electrode 3 can be moved along the longitudinal axis "A" by a rotation of the wheel 23 along the central axis "C". The adjusting device 4 also comprises a motor 24. Such motor 24 is preferably electric, more preferably an electromagnetic motor, and is installed on the wheel 23 in order to actuate the wheel 23 and through it, the electrode 3.

When the electrode 3 is consumable, the adjusting device 4 is configured for compensating the variation of distance between the electrode 3 and the weld area due to both the consumption of the electrode and the physiologic tremor of welder hand.

This double constant adjustment allows for maintaining a substantially constant distance between the electrode 3 and the weld area, allowing favorable arcing conditions and consequently an improved weld quality. With particular reference to the SMAW or MIG welding tool 2 of figure 2 please note that, in use, the wheel 23 advances overtime since the electrode 3 is consumed during welding. Therefore, the rotation speed of the motor 24 provides an overall forward movement to the electrode, and varies the rotation speed in order to adjust the distance of the tip of the electrode 3 as will be explained in a following part of the disclosure.

In the TIG welding tool 2 of figure 4 the electrode 3 is not consumed during welding. Therefore, the wheel 23 is moved only to adjust the distance of the electrode 3.

Also, in figure 4 a source of inert gas is present (not shown in the drawings) in order to shield the tip of the electrode 3 and the weld area from atmospheric oxygen. This source of inert gas is by itself known to the person skilled in the art, thus will not be described in detail.

The kit 1 comprises a voltage sensor 5 which is configured to detect a welding voltage "Vw" between the electrode 3 and the weld area, that is function of the distance between the end of the electrode facing the work piece and the weld area of the work piece. The voltage sensor 5 is also configured to emit a voltage signal "Vs", which is representative of a value of the welding voltage "Vw". Such voltage sensor 5 can be of any type known to the person skilled in the art, and therefore will not be described in detail.

The kit 1 also comprises a control unit 6. In the following part of the disclosure, the control unit 6 will be described by subdividing it into a plurality of modules. Such subdivision is done for ease of description only, and in no way should be considered as reflecting the physical structure of the control unit 6 itself. Rather, each module can be implemented as an electronic circuit on a suitable hardware support, as a software routine, subroutine or library or as both. Each module may reside on a local unit or may be distributed over a network. Also, the modules can communicate with each other either via a suitable wired or wireless protocol.

The control unit 6 comprises a data acquisition module 7, which is configured to acquire the above mentioned voltage signal "Vw".

The control unit 6 also comprises a memory module 16, which is configured to store a target voltage value "Vt".

The control unit 6 also comprises an input module 17 configured to set said target voltage value "Vt" in said memory module 16. In a particular embodiment of the invention the input module 17 can be a QR code reader. In this way the voltage "Vt", as well as any other parameter related to the welding process, can be read by the input module 17 on a suitably encoded QR code.

The control unit 6 also comprises a processing module 8, which is configured to output an actuation signal "Sa" function of at least the voltage signal "Vs". Also, the processing module 8 is configured to retrieve the target voltage value "Vt" and to compare it with the welding voltage value "Vw". The actuation signal "Sa" is therefore at least in part directly proportional to the result of such comparison. With additional detail, the processing module 8 may be programmed with a PID (Proportional, Integral and Derivative) logic. Therefore, the actuation signal "Sa" may be the sum of a part directly proportional to the difference between "Vw" and Vt", of a part proportional to the derivative of such difference and of a part proportional to the integral of such difference. Any possible combination can be used, depending on the chosen control strategy. The processing module 8 can also be configured to supply a voltage difference signal "Dv" representing the result of the difference between "Vw" and "Vt".

The control unit 6 also comprises an actuation module 14 connected to the adjusting device 4. The actuation module 14 is configured to operate the adjusting device 4 as directed by the actuation signal "Sa". In particular, the actuation module 14 operates the motor 24 which rotates the wheel 23. Optionally, the welding kit also comprises a welding mask 9. Such welding mask 9 is configured to be worn by a welder during a welding process as a standard safety mask. In particular, the welding mask 9 comprises a darkened window 10 from which the welder may observe the welding process without being blinded by the intense light.

Additionally, the welding mask 9 is provided with a welding velocity sensor 11. The welding velocity sensor 11 is configured to detect a welding velocity "Wa", and to emit a welding velocity signal "Ws" representing a value of the welding velocity "Wa".

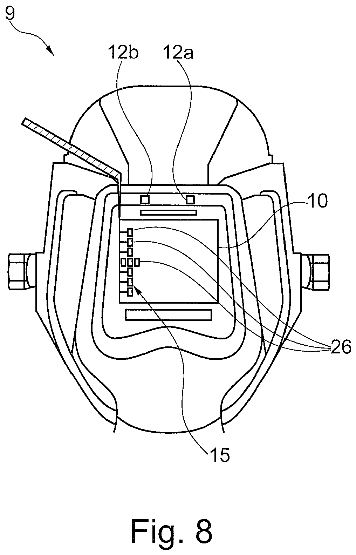

According to a preferred embodiment of the invention, the welding velocity sensor 11 comprises a first optical sensor 12a. The first optical sensor 12a is in particular arranged so that, during welding operation it faces the weld area. As shown in figure 8, the first optical sensor is preferably placed on the external surface of the welding mask 9, preferably over the darkened window 10. The welding velocity sensor 11 preferably also comprises a reference frame sensor 12b. This reference frame sensor 12b can be any kind of sensor which is able to detect a motion within a fixed frame of reference. For example, the reference frame sensor 12b can be an inertial sensor located on any point of the welding mask 9.

With more detail, in the embodiment shown in figure 8 the reference frame sensor 12b is a second optical sensor. The reference frame sensor 12b is therefore preferably arranged to face a fixed reference scene in the environment, as for example the work piece part from the weld area, and placed preferably beside the first optical sensor 12a. In the preferred embodiment of the invention, the sensors 12a, 12b are imaging cameras.

With additional detail, the first optical sensor 12a is configured to detect the velocity of the welding pool relative to itself. Also, the reference frame sensor 12b is configured to detect the velocity of the above mentioned fixed reference scene. According to a first embodiment, the welding velocity sensor 11 also comprises a velocity computing module 13 which is configured to compute the welding velocity "Wa" as a difference between the velocities detected by the second 12b and the first optical sensor 12a. Alternatively, the first optical sensor 12a and reference frame sensor 12b both transmit the respective velocities to the control unit 6, in particular to the data acquisition module 14.

The processing module 8 is also configured to compute a velocity difference between the welding velocity "Wa" and a target velocity "Wt" value, said processing unit being configured to emit a velocity difference signal "Dw" representing the result of said velocity difference.

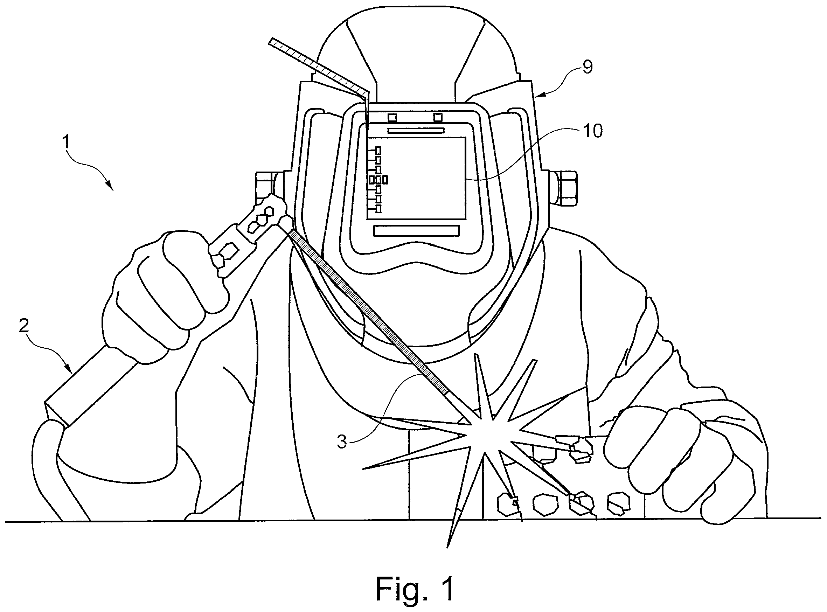

Optionally, the welding mask 9 comprises a visualization device 15. Such visualization device 15 is arranged to be easily visible by the welder during the welding process. As shown in figures 1 and 8, the visualization device 15 is preferably placed inside the welding mask 9, preferably on one side of the darkened window 10.

With more detail, the visualization device 15 is configured to acquire the above mentioned velocity difference signal "Dw", thus showing a representation of the velocity difference to the welder. Similarly, the visualization device 15 can be configured to acquire the voltage difference signal "Dv" mentioned above and to show a representation of the voltage difference to the welder.

Preferably, the visualization device 15 can be configured to show an operating parameter of the welding process, such as the voltage (V), the current (A), the welding speed (W), respectively between the electrode and the weld area, or their combinations. As shown schematically in figure 8, the visualization device comprises a plurality of LEDs 26. These LEDs are preferably arranged in a cross, and are configured to lighten in such a way as to indicate whether the welder should go faster or slower, or if he should get nearer or farther from the weld area.

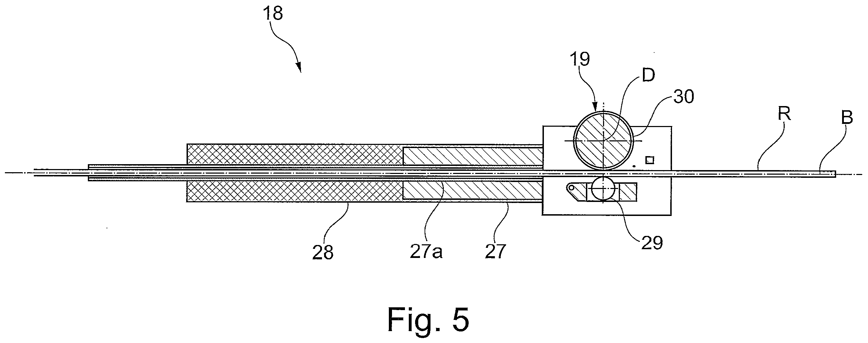

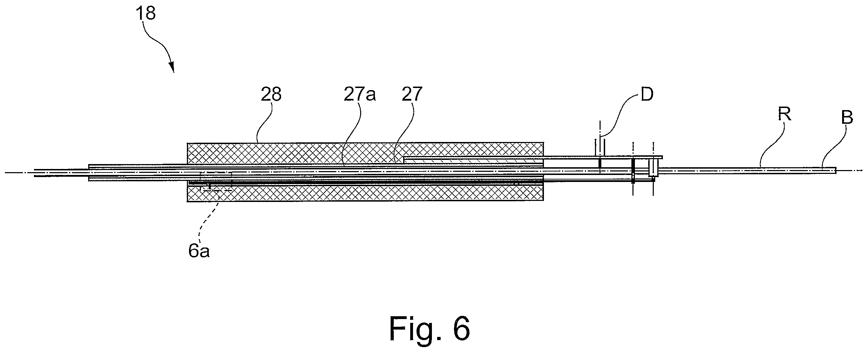

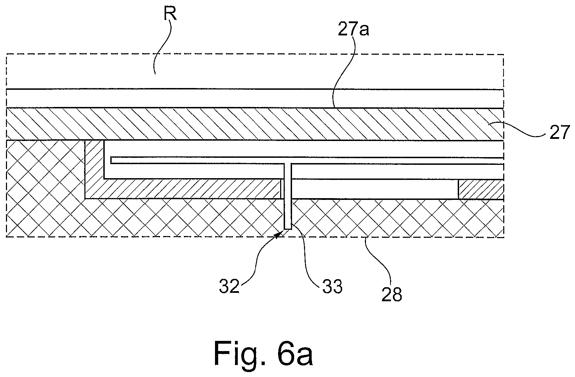

Referring specifically to figures 5 and 6, the kit 1 can also comprises a handling apparatus 18 for a filler rod "R". The handling apparatus 18 comprises a feeding device 19 configured to advance the filler rod "R" during welding.

With additional details, the handling apparatus 18 comprises a main body 27, configured to support the filler rod "R". The main body 27 is preferably axial-symmetric, and develops mainly along a longitudinal axis "B". A handle 28 for the welder is attached to the main body 27. Preferably, the handle 28 surrounds the main body 27 of the handling apparatus 18.

The main body 27 has a central seat 27a in which the filler rod "R" is placed. As shown in figure 5, the handling apparatus 18 is provided with bearings 29, which are attached to the main body 27 and located in proximity of the central seat 27a, to support the filler rod "R" and allow it to move forward/backward. In other words, the filler rod "R" can move forward/backward inside the seat 27a by sliding on the bearings 29.

The feeding device 19 comprises a wheel 30 having a central axis "D" disposed transversally, and preferably perpendicularly to the longitudinal axis "B" of the main body 27.

In operation, the rim of the wheel 30 is in contact with the filler rod "R" so that it can be moved along the longitudinal axis "B" by a rotation of the wheel 30 along the central axis "D". The feeding device 19 also comprises a motor 31. Such motor 31 is preferably electric, more preferably linear electromagnetic, and is installed on the wheel 30 in order to actuate the filler rod "R".

In an alternative embodiment, not shown in the drawings, the feeding device 19 comprises an electromagnetic actuation device for the filler rod "R" instead of the wheel 30 and the motor 31.

If the handling apparatus 18 is used, the processing module 8 may be configured to emit a feeding velocity signal "Sv" to the actuation module 14. The feeding velocity signal "Sv" is preferably proportional to a feeding velocity value "Fv". The actuation module 14 is therefore also configured to operate the feeding device 19 of the handling apparatus 18 as directed by the feeding velocity signal "Sv".

Also, as shown in figure 6a, the handling apparatus 18 comprises a control interface 32 associated with the processing module 8. The control interface 32 is configured to emit a command signal "Cv" to the processing module 8, so that the welder can increase or decrease the feeding velocity signal "Sa".

With additional detail, the control interface 32 comprises a button 33 placed on the handle 28. Specifically, the button 33 allows the welder to adjust the feeding velocity continuously; however the button 33 is designed as to give a tactile feedback in the form of "clicks" at predetermined intervals so that the welder can be made aware with a certain precision of the amount that the feeding velocity is being manually increased or decreased.

## Claims

1. A welding tool (2) comprising a main body (20); a handle (21) attached to said main body (20) and configured to be held by a welder; and an electrode (3); **characterized by** an adjusting device (4) attached to said main body (20) and associated with said electrode (3) for moving said electrode (3) forward/backward with respect to said main body (20); and a control unit (6) connected to said adjusting device (4) and configured to act on said adjusting device (4) for maintaining a substantially constant distance between said electrode (3) and a weld area; wherein said electrode (3) is consumable for performing a SMAW or a MIG weld, the welding tool (2) also comprising a voltage sensor (5) configured to detect a welding voltage (Vw) between said electrode (3) and said weld area and to emit a voltage signal (Vs) representing a value of said welding voltage (Vw), said voltage sensor (5) being connected to said control unit (6), wherein said control unit (6) comprises a data acquisition module (7) configured to acquire said voltage signal (Vw), a processing module (8) configured to output an actuation signal (Sa) function of at least said voltage signal (Vs), an actuation module (14) connected to said adjusting device (4) and configured to operate said adjusting device (4) as directed by said actuation signal (Sa).

2. A welding tool (2) according to the preceding claim, wherein the control unit (6) also comprises a memory module (16) configured to hold a target voltage value (Vt), said processing module (8) being configured to retrieve said target voltage value (Vt) and to compare it with said welding voltage value (Vw), said actuation signal (Sa) being at least in part directly proportional to the result of said comparison.

3. A welding tool (2) according to any preceding claim, wherein the control unit (6) also comprises an input module (17) configured to set said target voltage value (Vt) in said memory module (16).

4. A welding tool (2) according to claim 3, wherein the input module (17) is a QR code reader for reading said target voltage value (Vt) from a QR code.

5. A welding tool (2) according to any one of the previous claims, wherein said adjusting device (4) comprises a wheel (23) having a central axis (C) disposed transversally, and preferably perpendicularly, to a longitudinal axis (A) of the electrode (3), the rim of said wheel (23) being in contact with said electrode (3) for moving said electrode (3) forward/backward with respect to said main body (20).

6. A welding tool (2) according to the previous claim, wherein said adjusting device (4) comprises a motor (24), preferably electric, said motor (24) being connected to said wheel (23) for actuating said wheel (23).

7. A welding tool (2) according to the previous claim, wherein the motor (24) is a linear electromagnetic motor.

8. An arc welding kit (1) comprising a welding tool (2) according to any one of the previous claims; a welding mask (9); a visualization device (15) attached to said welding mask and arranged to show a representation of at least a performance of a welding operation selected from: welding speed (W), voltage (V), current (A), respectively between the electrode and the weld area.

9. An arc welding kit (1) according to the previous claim, wherein the welding mask (9) further comprises a welding velocity sensor (11) attached on the welding mask (9), said welding velocity sensor (11) being configured to detect a welding velocity (Wa) and to emit a welding velocity signal (Ws) representing a value of said welding velocity (Wa), said processing module (8) being configured to compute a velocity difference between said welding velocity (Wa) and a target velocity (Wt) value for said welding velocity, said processing module (8) being configured to emit a velocity difference signal (Dw) representing the result of said velocity difference; visualization device (15) being configured to acquire said velocity difference signal (Dw) and to show a representation of said velocity difference and of consequent heat input difference to a welder.

10. An arc welding kit (1) according to claim 8 or claim 9, wherein said processing module (8) is configured to compute a voltage difference between said welding voltage (Vw) and said target voltage (Vt), said processing module (8) being configured to emit a voltage difference signal (Dv) representing the result of said voltage difference; said visualization device (15) being configured to acquire said voltage difference signal (Dv) and to show a representation of said voltage difference to a welder.

## Patentansprüche

1. Schweißwerkzeug (2), umfassend einen Hauptkörper (20); einen Griff (21), der an dem Hauptkörper (20) befestigt und dazu ausgelegt ist, von einem Schweißer gehalten zu werden; und eine Elektrode (3); **gekennzeichnet durch** eine Einstellvorrichtung (4), die an dem Hauptkörper (20) angebracht und der Elektrode (3) zugeordnet ist, um die Elektrode (3) in Bezug auf den Hauptkörper (20) vorwärts/rückwärts zu bewegen; und eine Steuereinheit (6), die mit der Einstellvorrichtung (4) verbunden und dazu ausgelegt ist, um auf die Einstellvorrichtung (4) einzuwirken, um einen im Wesentlichen konstanten Abstand zwischen der Elektrode (3) und einem Schweißbereich aufrechtzuerhalten; wobei die Elektrode (3) verbrauchbar ist, um eine SMAW- oder eine MIG-Schweißung durchzuführen, wobei das Schweißwerkzeug (2) auch einen Spannungssensor (5) umfasst, der dazu ausgelegt ist, um eine Schweißspannung (Vw) zwischen der Elektrode (3) und dem Schweißbereich zu erfassen und ein Spannungssignal (Vs) auszugeben, das einen Wert der Schweißspannung (Vw) darstellt, wobei der Spannungssensor (5) mit der Steuereinheit (6) verbunden ist, wobei die Steuereinheit (6) ein Datenerfassungsmodul (7), das ausgelegt ist, um das Spannungssignal (Vw) zu erfassen, ein Verarbeitungsmodul (8), das ausgelegt ist, um eine Funktion eines Betätigungssignals (Sa) von mindestens dem Spannungssignal (Vs) auszugeben, ein Betätigungsmodul (14), das mit der Einstellvorrichtung (4) verbunden ist, umfasst und ausgelegt ist, um die Einstellvorrichtung (4) wie durch das Betätigungssignal (Sa) gesteuert zu betreiben.

2. Schweißwerkzeug (2) nach dem vorstehenden Anspruch, wobei die Steuereinheit (6) auch ein Speichermodul (16) umfasst, das ausgelegt ist, einen Spannungssollwert (Vt) zu halten, wobei das Verarbeitungsmodul (8) ausgelegt ist, um den Zielspannungswert (Vt) abzurufen und ihn mit dem Schweißspannungswert (Vw) zu vergleichen, wobei das Betätigungssignal (Sa) zumindest teilweise direkt proportional zu dem Ergebnis des Vergleichs ist.

3. Schweißwerkzeug (2) nach einem der vorstehenden Ansprüche, wobei die Steuereinheit (6) auch ein Eingabemodul (17) umfasst, das ausgelegt ist, um den Zielspannungswert (Vt) in dem Speichermodul (16) einzustellen.

4. Schweißwerkzeug (2) nach Anspruch 3, wobei das Eingabemodul (17) ein QR-Codeleser zum Lesen des Zielspannungswerts (Vt) von einem QR-Code ist.

5. Schweißwerkzeug (2) nach einem der vorstehenden Ansprüche, wobei die Einstellvorrichtung (4) ein Rad (23) mit einer Mittelachse (C) umfasst, die quer angeordnet ist, und sich vorzugsweise senkrecht zu einer Längsachse (A) der Elektrode (3) erstreckt, wobei der Rand des Rads (23) in Kontakt mit der Elektrode (3) steht, um die Elektrode (3) in Bezug auf den Hauptkörper (20) vorwärts/rückwärts zu bewegen.

6. Schweißwerkzeug (2) nach dem vorstehenden Anspruch, wobei die Einstellvorrichtung (4) einen Motor (24), vorzugsweise elektrisch, umfasst, wobei der Motor (24) mit dem Rad (23) verbunden ist, um das Rad (23) zu betätigen.

7. Schweißwerkzeug (2) nach dem vorstehenden Anspruch, wobei der Motor (24) ein elektromagnetischer Linearmotor ist.

8. Lichtbogenschweißset (1), umfassend ein Schweißwerkzeug (2) nach einem der vorstehenden Ansprüche; eine Schweißmaske (9); eine Visualisierungsvorrichtung (15), die an der Schweißmaske angebracht und angeordnet ist, um eine Darstellung mindestens einer Durchführung eines Schweißvorgangs zu zeigen, der ausgewählt ist aus: Schweißgeschwindigkeit (W), Spannung (V), Strom (A), jeweils zwischen der Elektrode und dem Schweißbereich.

9. Lichtbogenschweißset (1) nach dem vorstehenden Anspruch, wobei die Schweißmaske (9) ferner einen Schweißgeschwindigkeitssensor (11) umfasst, der an der Schweißmaske (9) angebracht ist, wobei der Schweißgeschwindigkeitssensor (11) ausgelegt ist, um eine Schweißgeschwindigkeit (Wa) zu erfassen und ein Schweißgeschwindigkeitssignal (Ws) auszugeben, das einen Wert der Schweißgeschwindigkeit (Wa) darstellt, wobei das Verarbeitungsmodul (8) ausgelegt ist, um eine Geschwindigkeitsdifferenz zwischen der Schweißgeschwindigkeit (Wa) und einem Zielgeschwindigkeitswert (Wt) für die Schweißgeschwindigkeit zu berechnen, wobei das Verarbeitungsmodul (8) ausgelegt ist, um ein Geschwindigkeitsdifferenzsignal (Dw) auszugeben, welches das Ergebnis der Geschwindigkeitsdifferenz darstellt; Visualisierungsvorrichtung (15), die ausgelegt ist, um das Geschwindigkeitsdifferenzsignal (Dw) zu erfassen und einem Schweißgerät eine Darstellung der Geschwindigkeitsdifferenz und der daraus folgenden Wärmeeingangsdifferenz anzuzeigen.

10. Lichtbogenschweißset (1) nach Anspruch 8 oder Anspruch 9, wobei das Verarbeitungsmodul (8) ausgelegt ist, um eine Spannungsdifferenz zwischen der Schweißspannung (Vw) und der Zielspannung (Vt) zu berechnen, wobei das Verarbeitungsmodul (8) ausgelegt ist, um ein Spannungsdifferenzsignal (Dv) auszugeben, welches das Ergebnis der Spannungsdifferenz darstellt; wobei die Visualisierungsvorrichtung (15) ausgelegt ist, um das Spannungsdifferenzsignal (Dv) zu erfassen und einem Schweißgerät eine Darstellung der Spannungsdifferenz anzuzeigen.

## Revendications

1. Outil de soudage (2) comprenant un corps principal (20) ; une poignée (21) fixée audit corps principal (20) et configurée pour être maintenue par un soudeur ; et une électrode (3) ; **caractérisé par** un dispositif de réglage (4) fixé audit corps principal (20) et associé à ladite électrode (3) pour déplacer ladite électrode (3) vers l'avant/vers l'arrière par rapport audit corps principal (20) ; et une unité de commande (6) reliée audit dispositif de réglage (4) et configurée pour agir sur ledit dispositif de réglage (4) pour maintenir une distance sensiblement constante entre ladite électrode (3) et une zone de soudure ; dans laquelle ladite électrode (3) est consommable pour exécuter une soudure SMAW ou MIG, l'outil de soudage (2) comprenant également un capteur de tension (5) configuré pour détecter une tension de soudage (Vw) entre ladite électrode (3) et ladite zone de soudure et pour émettre un signal de tension (Vs) représentant une valeur de ladite tension de soudure (Vw), ledit capteur de tension (5) étant relié à ladite unité de commande (6), dans lequel ladite unité de commande (6) comprend un module d'acquisition de données (7) configuré pour acquérir ledit signal de tension (Vw), un module de traitement (8) configuré pour produire une fonction de signal d'actionnement (Sa) d'au moins ledit signal de tension (Vs), un module d'actionnement (14) relié audit dispositif de réglage (4) et configuré pour actionner ledit dispositif de réglage (4) tel que dirigé par ledit signal d'actionnement (Sa).

2. Outil de soudage (2) selon la revendication précédente, dans lequel l'unité de commande (6) comprend également un module de mémoire (16) configuré pour maintenir une valeur de tension cible (Vt), ledit module de traitement (8) étant configuré pour récupérer ladite valeur de tension cible (Vt) et pour la comparer à ladite valeur de tension de soudure (Vw), ledit signal d'actionnement (Sa) étant au moins en partie directement proportionnel au résultat de ladite comparaison.

3. Outil de soudage (2) selon une quelconque revendication précédente, dans lequel l'unité de commande (6) comprend également un module d'entrée (17) configuré pour régler ladite valeur de tension cible (Vt) dans ledit module de mémoire (16).

4. Outil de soudage (2) selon la revendication 3, dans lequel le module d'entrée (17) est un lecteur de code QR pour lire ladite valeur de tension cible (Vt) à partir d'un code QR.

5. Outil de soudage (2) selon l'une quelconque des revendications précédentes, dans lequel ledit dispositif de réglage (4) comprend une roue (23) ayant un axe central (C) disposé transversalement, et de préférence perpendiculairement, à un axe longitudinal (A) de l'électrode (3), la jante de ladite roue (23) étant en contact avec ladite électrode (3) pour déplacer ladite électrode (3) vers l'avant/vers l'arrière par rapport audit corps principal (20).

6. Outil de soudage (2) selon la revendication précédente, dans lequel ledit dispositif de réglage (4) comprend un moteur (24), de préférence électrique, ledit moteur (24) étant relié à ladite roue (23) pour actionner ladite roue (23).

7. Outil de soudage (2) selon la revendication précédente, dans lequel le moteur (24) est un moteur électromagnétique linéaire.

8. Kit de soudage à l'arc (1) comprenant un outil de soudage (2) selon l'une quelconque des revendications précédentes ; un masque de soudage (9) ; un dispositif de visualisation (15) fixé audit masque de soudage et agencé pour montrer une représentation d'au moins une performance d'une opération de soudage choisie parmi : la vitesse de soudage (W), la tension (V), le courant (A), respectivement entre l'électrode et la zone de soudure.

9. Kit de soudage à l'arc (1) selon la revendication précédente, dans lequel le masque de soudage (9) comprend en outre un capteur de vitesse de soudage (11) fixé sur le masque de soudage (9), ledit capteur de vitesse de soudage (11) étant configuré pour détecter une vitesse de soudage (Wa) et pour émettre un signal de vitesse de soudage (Ws) représentant une valeur de ladite vitesse de soudage (Wa), ledit module de traitement (8) étant configuré pour calculer une différence de vitesse entre ladite vitesse de soudage (Wa) et une valeur de vitesse cible (Wt) pour ladite vitesse de soudage, ledit module de traitement (8) étant configuré pour émettre un signal de différence de vitesse (Dw) représentant le résultat de ladite différence de vitesse ; un dispositif de visualisation (15) étant configuré pour acquérir ledit signal de différence de vitesse (Dw) et pour montrer une représentation de ladite différence de vitesse et de la différence d'entrée de chaleur conséquente à un soudeur.

10. Kit de soudage à l'arc (1) selon la revendication 8 ou la revendication 9, dans lequel ledit module de traitement (8) est configuré pour calculer une différence de tension entre ladite tension de soudage (Vw) et ladite tension cible (Vt), ledit module de traitement (8) étant configuré pour émettre un signal de différence de tension (Dv) représentant le résultat de ladite différence de tension ; ledit dispositif de visualisation (15) étant configuré pour acquérir ledit signal de différence de tension (Dv) et pour montrer une représentation de ladite différence de tension à un soudeur.
